# EUROPÄISCHE PATENTANMELDUNG

(11) **EP 2 790 019 A1**
(43) Veröffentlichungstag der Anmeldung: **15.10.2014**
(21) Anmeldenummer: 13163026.1
(22) Anmeldetag: 10.04.2013
(51) Int. Cl.: G01N 33/557

(54) **High Dose Hook Erkennung**

(71) Anmelder: Siemens Healthcare Diagnostics Products GmbH, 35041 Marburg (DE)
(72) Erfinder: Balke, Thomas, 35085 EBSDORFERGRUND (DE); Uelker, Ugur, 35041 Marburg (DE)

(57) **Zusammenfassung**

Verfahren zur Bestimmung der Konzentration oder Aktivität eines Analyten, welches die Erkennung und/oder den Ausschluss des Vorliegens des High Dose Hook Effektes durch Messung und Analyse der Reaktionskinetik ermöglicht.

## Beschreibung

Die Erfindung liegt auf dem Gebiet der Analysetechnik und betrifft ein Verfahren zur High Dose Hook Erkennung bei der Bestimmung der Konzentration oder Aktivität eines Analyten in einer Probe.

In der modernen Analysetechnik kommt je nach Art des zu bestimmenden Analyten eine Vielzahl von unterschiedlichen Verfahrensprinzipien zum Einsatz. Um zum Beispiel Proteine oder Peptide quantitativ nachzuweisen oder qualitativ zu bestimmen, werden bevorzugt immunochemische Verfahren angewandt. Dazu werden üblicherweise Antikörper verwendet, die den Analyten spezifisch erkennen. Um besonders aussagekräftige und genaue Ergebnisse zu erhalten, ist es häufig erforderlich, eine Nachweisreaktion über einen Zeitraum zu beobachten und ihren Verlauf zu analysieren. Die Veränderung einer reaktionsabhängigen Messgröße über die Zeit wird auch als Reaktionskinetik oder Signal-Zeit-Kurve bezeichnet. Verschiedene Parameter einer solchen Kurve können zur Auswertung und damit Bestimmung der Konzentration oder Aktivität eines Analyten verwendet werden.

Beispiele für Nachweisreaktionen, deren Reaktionskinetik ausgewertet wird, sind z.B. partikelverstärkende Agglutinationsassays, bei denen beispielsweise partikelgebundene Antikörper oder Antikörperfragmente mit einem Antigen reagieren, wodurch es zur Bildung von stark lichtstreuenden Molekülaggregaten kommt. Die analytabhängige Bildung dieser Molekülaggregate kann über die Messung von Streulichtintensität (Nephelometrie) oder über die Messung von Absorption (Turbidimetrie) gemessen und zur Bestimmung der Konzentration des Analyten genutzt werden. Weitere Beispiele sind enzymbasierte Testverfahren, bei denen die Umsetzung z.B. eines chromogenen Substrates über die Zeit gemessen wird. Die analytabhängige Spaltung eines chromogenen Substrates kann z.B. über die Messung der Absorption bei einer bestimmten Wellenlänge gemessen werden und zur Bestimmung der Konzentration oder Aktivität des Analyten genutzt werden. Ein anderes Beispiel sind Gerinnungstests, bei denen die Aktivität eines einzelnen oder mehrerer Blutgerinnungsfaktoren z.B. durch die Messung der Fibrinbildungsgeschwindigkeit in einer Blut- oder Plasmaprobe bestimmt wird.

Verschiedene Parameter einer Reaktionskinetik (Signal-Zeit-Kurve) können für eine Auswertung verwendet werden. Bekannte Parameter sind z.B. die maximale Reaktionsgeschwindigkeit bzw. der Zeitpunkt des Auftretens der maximalen Reaktionsgeschwindigkeit. Häufig wird zur Auswertung zunächst die erste oder zweite Ableitung der Kinetik gebildet. Ein weiterer bekannter Parameter ist z.B. die Fläche unter der ersten Ableitung einer Signal-Zeit-Kurve. In anderen Fällen wird der Zeitpunkt bestimmt, bei dem das Signal einen vorbestimmten Schwellenwert überschreitet oder unterschreitet, oder es wird die Signalhöhe bestimmt, die zu einem vorbestimmten Zeitpunkt erreicht wird. Der übrige Verlauf der Reaktionskurve wird dann in erster Linie zur Überprüfung der Plausibilität der Messwerte ausgewertet.

Im einfachsten Fall eines Verfahrens zur Bestimmung der Konzentration oder der Aktivität eines Analyten wird eine Probe, die den Analyten vermutlich enthält, mit einem Reagenz vermischt, das die zum Nachweis des Analyten notwendigen Komponenten, wie z.B. ein Substrat, einen Bindungspartner, einen Aktivator oder ähnliches enthält. Üblicherweise wird die Reaktion ab Zugabe des Reagenzes, d.h. ab dem Zeitpunkt t0 anhand der Messung eines geeigneten physikalischen Signals verfolgt. Idealerweise verhält sich das Messsignal zu jedem Zeitpunkt proportional zur Reaktion. Tatsächlich kommt es jedoch zu verschiedenen Phänomenen, wie z.B. Ungenauigkeiten des Messinstruments, die dazu führen, dass sich nicht jedes absolute Messsignal proportional zur Reaktion verhält. Ferner kann es zu systematischen Effekten wie z.B. dem High Dose Hook Effekt kommen.

Der High Dose Hook Effekt ist dadurch charakterisiert, dass hohe Konzentrationen des Analyten in der Probe, die typischerweise deutlich über der Konzentration des höchsten Kalibrators des Assays liegen, ein falsch-niedriges Ergebnis im Assay zeigen. Der High Dose Hook Effekt wird vorwiegend bei Einschritt-Two-site (sandwich) Immunoassays beobachtet, weniger bei Zweischritt-Assays. Bei den Einschritt-Assays sind z.B. Fänger-Antikörper, Analyt und markierter Antikörper gleichzeitig im Reaktionsansatz. Beim Zweischritt-Assay wird im ersten Schritt nur mit der Probe inkubiert und der Analyt bindet an den Fänger-Antikörper. Dann wird überschüssiger Analyt durch Waschen entfernt und in einem zweiten Schritt markierter Antikörper hinzugegeben.

Beim Einschritt-Enzymimmunoassay soll der High Dose Hook Effekt darauf beruhen, dass nach Sättigung des Fänger-Antikörpers durch den Analyten überschüssiger Analyt z.B. an den enzymmarkierten Antikörper bindet und diesen blockiert, so dass sich kein Sandwich ausbilden kann. Es resultiert letztendlich ein Signalverlust mit der Folge, dass die gemessene Analytkonzentration niedriger ist als die wirkliche.

Bei Agglutinationsverfahren soll der High Dose Hook Effekt darauf beruhen, dass ein Überangebot von Antigen die Bindungsstellen des Antikörpers absättigt, so dass es zu einer geringeren, oder keiner Vernetzung bzw. Verklumpung kommt. Es entstehen daher weniger oder keine über z.B. Streulichtmessverfahren messbare Komplexe.

Bei dem selten auftretenden High Dose Hook Effekt der Zweischritt-Assays soll die Ursache auf multiplen Interaktionen zwischen dem Fänger-Antikörper-gebundenen Analyten und dem markierten Antikörper beruhen. Dabei soll es zu Konformationsänderungen des Analyten und dessen Freisetzung vom Fänger-Antikörper kommen.

Beim High Dose Hook Effekt führt die sukzessive Verdünnung der Probe zunehmend zu höheren anstatt zu niedrigeren Ergebnissen bis die Konzentration die Äquivalenzzone erreicht. Analyten, bei denen der High Dose Hook Effekt häufig beschrieben ist, sind z.B. Calcitonin, hCG, AFP, CA 125, Ferritin, PSA, Prolactin, TSH.

Die Begriffe "High Dose Hook Effekt", "Hook Effekt", "Prozone Effekt" sowie "Antigen Excess Effekt" werden synonym verwendet.

Im Stand der Technik werden verschiedene Ansätze beschrieben, um das Vorliegen des High Dose Hook Effektes in einer Probe zu erkennen, um entsprechende Messergebnisse z.B. markieren, verwerfen oder speziell weiter auswerten zu können.

WO 2010/004416 A2 beschreibt ein Verfahren, welches das Erkennen des Vorliegens des High Dose Hook Effektes in einer Probe durch Auswertung der Reaktionskinetik zu Beginn der immunochemischen Reaktion ermöglicht. In der Praxis zeigt sich jedoch, dass dieses Verfahren nicht immer funktioniert und z.B. ein Vorliegen des High Dose Hook Effektes in einer Probe mittels dieses Verfahrens bei einer nephelometrischen Messung der Immunglobulin A Bestimmung nicht zuverlässig ermittelt werden kann.

Hoffman, K. L., et al. "Elimination of "hook-effect" in two-site immunoradiometric assays by kinetic rate analysis", Clinical Chemistry 30(9), (1984): 1499-1501, beschreibt ein Verfahren zur Eliminierung des High Dose Hook Effektes in immunoradioaktiven Assays zur Bestimmung von humanem Choriogonadotropin (hCG) durch Analyse der Reaktionskinetik bei gleichzeitiger Inkubation der Festphase und des Antikörpers. Die Analyse der Reaktionskinetik erfolgt durch Auswertung von Signalen und Berechung der korrespondierenden scheinbaren Konzentrationen bei 2 Minuten nach Beginn der Reaktion, und bei 10 Minuten nach Beginn der Reaktion. Die so ermittelten Konzentrationen werden ins Verhältnis gesetzt. Überschreitet das Verhältnis einen Schwellenwert, so wird auf das Vorliegen des High Dose Hook Effektes geschlossen. Nachteilig ist bei diesem Verfahren, dass das Vorliegen des High Dose Hook Effektes erst gegen Ende der immunochemischen Reaktion vorliegt, dass Messungen zu zwei Zeitpunkten benötigt werden, und die Messpunkte mit 8 Minuten zeitlich weit auseinander liegen. Ferner ist nachteilig, dass je eine Referenzkurve für die Konzentrationsbestimmung bei 2 Minuten und bei 10 Minuten nach Beginn der Reaktion hinzugezogen werden müssen, um die jeweiligen Konzentrationen zu ermitteln.

Papik, Kornel, et al. "Automated prozone effect detection in ferritin homogeneous immunoassays using neural network classifiers", Clinical Chemistry and Laboratory Medicine 37(4), (1999): 471-476, beschreibt ein Verfahren zur Erkennung des Vorliegens des High Dose Hook Effektes durch Analyse der Reaktionskinetik nach der Messung. Das Verfahren nutzt ein trainiertes virtuelles neuronales Netzwerk basierend auf 77 Proben von 48 Patienten, um die falsch-negativen Ergebnisse direkt nach der Bestimmung zu filtern. Das Verfahren nutzt dabei zahlreiche Reaktionskinetikkurven von Proben am Ende der Reaktion, bei denen der High Dose Hook Effekt auftritt. Nachteilig ist bei diesem Verfahren dessen große Komplexität und Aufwändigkeit. Das Verfahren benötigt die Bereitstellung zahlreicher Standardproben von zahlreichen Patienten zur Trainierung des neuronalen Netzwerkes. Die einmal ermittelten Kontrollparameter werden im System zeitlich konstant verwendet, auch unter z.B. signifikant veränderten Temperaturbedingungen, als die unter denen die Standardproben gemessen wurden. Dies kann die Verlässlichkeit des Verfahrens stark einschränken.

Der vorliegenden Erfindung liegt die Aufgabe zugrunde, ein alternatives Verfahren zur Bestimmung der Konzentration oder Aktivität eines Analyten bereit zu stellen, das die Erkennung und/oder den Ausschluss des Vorliegens des High Dose Hook Effektes durch Messung und Analyse der Reaktionskinetik ermöglicht und damit eine verlässliche Bestimmung der Konzentration oder Aktivität eines Analyten in einer Probe gewährleistet.

**Die Aufgabe wird erfindungsgemäß durch die im Folgenden beschriebenen Verfahren und Gegenstände gelöst.**

Ein bevorzugter Gegenstand der vorliegenden Erfindung ist ein Verfahren zur Bestimmung der Konzentration oder Aktivität eines Analyten in einer Probe, welches folgende Schritte umfasst:
a. Vermischen der Probe mit mindestens einem Reagenz, wodurch eine analytabhängige Reaktion in Gang gesetzt wird;
b. Messung eines sich infolge der analytabhängigen Reaktion ändernden Signals (x) über die Zeit (t);
c. Bestimmung der Steigung A der Signal-Zeit-Kurve in mindestens einem Zeitintervall;
d. Vergleich der Steigung A mit der ermittelten Steigung der Signal-Zeit-Kurve einer Referenzprobe mit einer definierten Analytkonzentration oder Analytaktivität oberhalb der Schwelle zum Eintritt des High Dose Hook Effekts, und / oder mit der ermittelten Steigung der Signal-Zeit-Kurve einer Referenzprobe mit einer definierten Analytkonzentration oder Analytaktivität unterhalb der Schwelle zum Eintritt des High Dose Hook Effekts;
e. Bestimmung der Konzentration oder Aktivität des Analyten anhand mindestens eines Parameters der Signal-Zeit-Kurve, **wobei**
die Bestimmung der Steigung A der Signal-Zeit-Kurve innerhalb eines Zeitraums beginnend 2 Minuten und endend 30 Minuten nach in Gangsetzung der analytabhängigen Reaktion durch Vermischen der Probe mit mindestens einem Reagenz erfolgt, und wobei
i. das Vorliegen des High Dose Hook Effekts verifiziert oder wahrscheinlich ist, wenn die Steigung A der Signal-Zeit-Kurve gleich oder größer ist als die Steigung der Referenzprobe mit einer definierten Analytkonzentration oder Analytaktivität oberhalb der Schwelle zum Eintritt des High Dose Hook Effekts, und / oder
ii. das Vorliegen des High Dose Hook Effekts ausgeschlossen oder unwahrscheinlich ist, wenn die Steigung A der Signal-Zeit-Kurve gleich oder kleiner ist als die Steigung der Referenzprobe mit einer definierten Analytkonzentration oder Analytaktivität unterhalb der Schwelle zum Eintritt des High Dose Hook Effekts.

Die Messung des sich infolge der analytabhänigen Reaktion ändernden Signals (x) über die Zeit (t) erfolgt mit Hilfe geeigneter Detektorvorrichtungen. Bei dem Signal kann es sich um jede dem Fachmann bekannte Signalart handeln, wie z.B. um optische Eigenschaften des Reaktionsansatzes, beispielsweise Trübung, Lichtstreuung, Fluoreszenz, Chemilumineszenz, um chemische Eigenschaften des Reaktionsansatzes, beispielsweise pH-Wert, Redoxpotential, Viskosität, Festigkeit, oder um elektronische Eigenschaften, beispielsweise Leitfähigkeit, Impedanz.

Die Messung über die Zeit (t) erfolgt beispielsweise durch die kontinuierliche Erfassung des physikalischen Signals, bevorzugt in gleichmäßigen Zeitabständen, wie z.B. eine Messung pro Sekunde, oder eine Messung pro 10 Sekunden. Alternativ erfolgt die Messung über die Zeit (t) beispielsweise durch die Erfassung des physikalischen Signals zu einem oder mehreren diskreten Zeitpunkten. Die so erhaltene Zeitkurve wird gespeichert, um der nachfolgenden Auswertung zur Verfügung zu stehen. Der Begriff "Signal-Zeit-Kurve" ist in diesem Zusammenhang breit zu verstehen und nicht auf die graphische Darstellung einer entsprechenden Kurve beschränkt. Vielmehr ist damit die Zuordnung der Messwerte zu den dazugehörigen Zeitpunkten in einer geordneten Reihe gemeint.

Die Bestimmung der Konzentration oder Aktivität des Analyten in der Probe erfolgt anhand mindestens eines Parameters der Signal-Zeit-Kurve.

Besonders bevorzugt eignet sich das erfindungsgemäße Verfahren falls sich (I) die Steigung A der Signal-Zeit-Kurve von Proben mit einer Analytkonzentration oder Analytaktivität unterhalb der Schwelle zum Eintritt des High Dose Hook Effekts nach den ersten 30 Sekunden zunehmend mit fortschreitender Zeit nach in Gangsetzung der analytabhängigen Reaktion durch Vermischen der Probe mit mindestens einem Reagenz verringert, und falls sich (II) die Steigung A der Signal-Zeit-Kurve von Proben mit einer Analytkonzentration oder Analytaktivität oberhalb der Schwelle zum Eintritt des High Dose Hook Effekts zunehmend mit fortschreitender Zeit nach 30 Sekunden nach in Gangsetzung der analytabhängigen Reaktion durch Vermischen der Probe mit mindestens einem Reagenz und bis einem Zeitpunkt, bei dem Proben mit einer Analytkonzentration oder Analytaktivität unterhalb der Schwelle zum Eintritt des High Dose Hook Effekts Sättigung oder Nahezu-Sättigung der Reaktion erreichen, vergrößert.

Hierbei ist zur Vermeidung von statistischen Abweichungen bei der Bestimmung der Steigung A über hinreichend große Zeitinterwalle zu mitteln.

Die Bestimmung der Steigung A der Signal-Zeit-Kurve erfolgt dabei bevorzugt innerhalb eines Zeitraums beginnend mit dem Ende des überdurchschnittlich ansteigenden Startbereichs und endend mit Erreichen der Sättigung oder Nahezu-Sättigung der analytabhängigen Reaktion einer Referenzprobe mit einer definierten Analytkonzentration oder Analytaktivität unterhalb der Schwelle zum Eintritt des High Dose Hook Effekts.

Unter dem Begriff "überdurchschnittlich ansteigender Startbereich" ist der Bereich der Signal-Zeit-Kurve ab ca. 30 Sekunden nach in Gangsetzung der analytabhängigen Reaktion durch Vermischen der Probe mit mindestens einem Reagenz zu verstehen, der bei Proben mit einer Analytkonzentration oder Analytaktivität unterhalb der Schwelle zum Eintritt des High Dose Hook Effekts eine Steigung A der Signal-Zeit-Kurve aufweist, die größer ist als die durchschnittliche Steigung der Signal-Zeit-Kurve gemittelt über einen Zeitraum beginnend ca. 30 Sekunden nach in Gangsetzung der analytabhängigen Reaktion durch Vermischen der Probe mit mindestens einem Reagenz und endend mit Erreichen der Sättigung oder Nahezu-Sättigung der Reaktion von Proben mit einer Analytkonzentration oder Analytaktivität unterhalb der Schwelle zum Eintritt des High Dose Hook Effekts.

Die jeweiligen Zeiten nach in Gangsetzung der analytabhängigen Reaktion durch Vermischen der Probe mit mindestens einem Reagenz hängen von der jeweiligen analytabhängigen Reaktion und weiteren Parametern wie z.B. Analytkonzentration, Temperatur, Druck, etc. ab und müssen mittels Referenzproben mit definierter Analytkonzentration oder Analytaktivität unterhalb der Schwelle zum Eintritt des High Dose Hook Effekts jeweils testspezifisch ermittelt werden.

Unter "Referenzprobe mit einer Analytkonzentration oder Analytaktivität unterhalb der Schwelle zum Eintritt des High Dose Hook Effekts" ist hier besonders bevorzugt jeweils die Referenzprobe mit der höchsten Analytkonzentration oder Analytaktivität noch unterhalb der Schwelle zum Eintritt des High Dose Hook Effekts zu verstehen.

In einer bevorzugten Ausführung erfolgt die Bestimmung der Steigung A der Signal-Zeit-Kurve innerhalb eines Zeitraums von 3 Minuten bis 20 Minuten, besonders bevorzugt von 4 bis 10 Minuten, besonders bevorzugt von 4 bis 7 Minuten, ganz besonders bevorzugt von 5 bis 6 Minuten nach in Gangsetzung der analytabhängigen Reaktion durch Vermischen der Probe mit mindestens einem Reagenz.

In einer bevorzugten Ausführungsform wird die Steigung A der Signal-Zeit-Kurve mit Hilfe einer linearen Regressionsrechnung bestimmt.

Unter einer Probe" ist im Sinne der Erfindung das Material zu verstehen, das die nachzuweisende Substanz, d.h. den Analyten, vermutlich enthält. Der Begriff "Probe" umfasst beispielsweise biologische Flüssigkeiten oder Gewebe insbesondere von Menschen und Tieren wie Blut, Plasma, Serum, Speichel, Sputum, Exudat, bronchoalveoläre Lavage, Lymphflüssigkeit, Synovialflüssigkeit, Samenflüssigkeit, Vaginalschleim, Faeces, Urin, Liquor, Haare, Haut, Gewebeproben oder -schnitte. Ferner sind umfasst Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfernen. Solche Vorbehandlungen von Proben mag die Abtrennung und / oder Lyse von Zellen beinhalten, das Präzipitieren, die Hydrolyse oder die Denaturierung von Probenbestandteilen wie z.B. Proteinen, die Zentrifugation von Proben, die Behandlung von Proben mit organischen Lösungsmitteln wie z.B. Alkohole, insbesondere Methanol; das Behandeln der Proben mit Detergenzien. Häufig wird die Probe in ein anderes, meistens wässriges Medium überführt, welches mit dem Nachweisverfahren möglichst nicht interferieren soll.

Unter dem Begriff "Analyt" ist im Sinne der Erfindung die nachzuweisende Substanz zu verstehen (Beispiele für den Begriff "Analyt" siehe EP 515 194 A2, Seiten 8 - 15) oder auch ein Medikament, oder auch ein biologischer Parameter, der Aufschluss über einen Zustand eines physiologischen und / oder pathologischen Prozesses bzw. eines mehrstufigen, multifaktoriellen Reaktionssystems gibt. Beispiele für derartige biologische Parameter sind z.B. die Konzentration von Immunglobulin A oder Immunglobulin-Leichtkette Typ Kappa, oder auch z.B. die verschiedenen Gerinnungszeiten, wie z.B. die Prothrombinzeit (PT), oder die aktivierte partielle Thromboplastinzeit (APTT).

In bevorzugter Ausführungsform des erfindungsgemäßen Verfahrens handelt es sich bei der analytabhängigen Reaktion um eine Antigen-Antikörper-Reaktion.

In bevorzugter Ausführungsform des erfindungsgemäßen Verfahrens wird dieses zur Bestimmung der Konzentration von Immunglobulin A oder Immunglobulin-Leichtkette Typ Kappa oder Immunglobulin-Leichtkette Typ Lambda angewandt.

In bevorzugter Ausführungsform des erfindungsgemäßen Verfahrens wird dieses zur Bestimmung der Konzentration oder Aktivität eines Analyten in einer biologischen Probe aus der Gruppe Vollblut, Plasma, Serum, Urin, Faeces, Speichel oder Liquor angewandt.

Ein weiterer Gegenstand der Erfindung ist ein Gerät, das imstande ist, das erfindungsgemäße Verfahren zur Bestimmung der Konzentration oder der Aktivität eines Analyten durchzuführen. Ein solches Gerät zeichnet sich dadurch aus, dass es über Mittel zum Vermischen der Probe mit mindestens einem Reagenz verfügt, wodurch eine analytabhängige Reaktion in Gang gesetzt wird, über Mittel zur Messung des sich infolge der analytabhängigen Reaktion ändernden Signals (x) über die Zeit (t), beispielsweise ein Nephelometer, Turbidimeter, Photometer, pH-Meter, Dosimeter, Luminometer, Fluorimeter oder ähnliches, über Mittel zur Bestimmung der Steigung A der Signal-Zeit-Kurve, beispielsweise Software, Computerprogramm, Algorithmus, sowie über Mittel zur Steuerung der Durchführung der weiteren Verfahrensschritte der erfindungsgemäßen Verfahren, beispielsweise Software, Computerprogramm, Algorithmus. Bevorzugterweise verfügt das Gerät auch über Mittel zur Ausgabe von Messergebnissen, beispielsweise elektronisches Anzeigegerät, Monitor, Datenschreiber, Drucker, und / oder Datenfernleitung. Bevorzugterweise verfügt das Gerät ferner über Mittel zur Speicherung der Signal-Zeit-Kurve, beispielsweise einen Halbleiterspeicher, ein optisches oder magnetisches Speichermedium wie etwa eine Festplatte.

Ein weiterer erfindungsgemäßer Gegenstand ist ein Speichermedium auf dem ein Programm zur Steuerung der Durchführung eines erfindungsgemäßen Verfahrens zur Bestimmung der Konzentration oder Aktivität eines Analyten in einer Probe abgespeichert ist.

Die in den Figuren beispielhaft dargestellten Ausführungsformen sollen die vorliegende Erfindung veranschaulichen und sind nicht als Einschränkung zu verstehen.

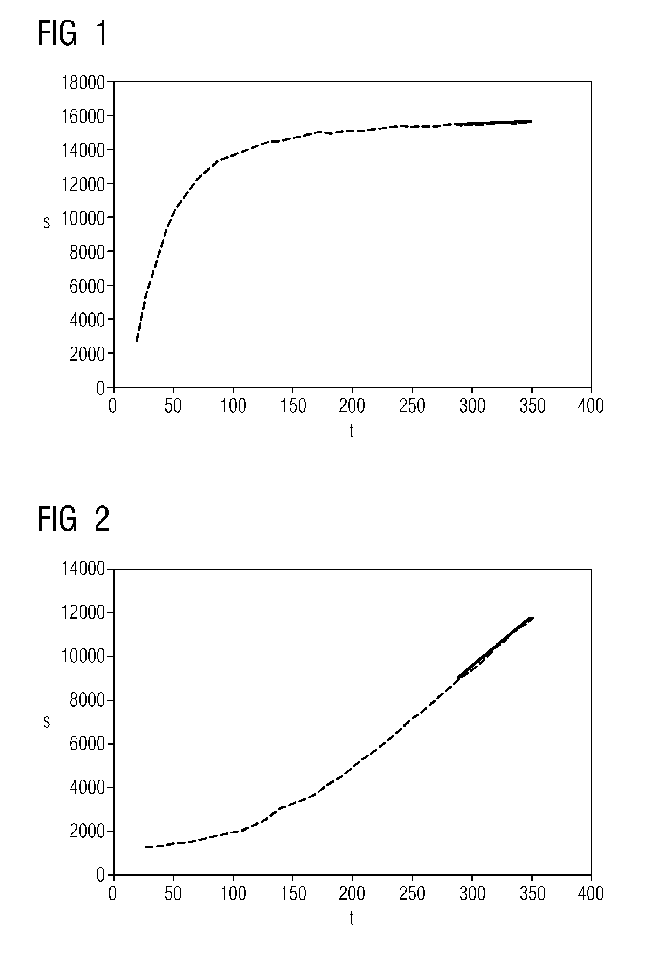

**Figur 1** zeigt den Verlauf einer Signal-Zeit-Kurve einer Antigen-Antikörper-Reaktion eines Agglutinationsverfahrens zur Bestimmung der Immunglobulin A Konzentration in einer Probe mit einer Immunglobulin A Konzentration unterhalb der Konzentrationsschwelle ab der der High Dose Hook Effekt auftritt. Die Steigung der Signal-Zeit-Kurve im Bereich von 277 bis 354 Sekunden beträgt 2,7 Signal-Einheiten/ Zeit-Einheiten. Das Signal wurde mittels eines Streulichtmessverfahrens bestimmt.

**Figur 2** zeigt den Verlauf einer Signal-Zeit-Kurve einer Antigen-Antikörper-Reaktion eines Agglutinationsverfahrens zur Bestimmung der Immunglobulin A Konzentration in einer Probe mit einer Immunglobulin A Konzentration oberhalb der Konzentrationsschwelle ab der der High Dose Hook Effekt auftritt. Die Steigung der Signal-Zeit-Kurve im Bereich von 277 bis 354 Sekunden beträgt 46 Signal-Einheiten/Zeit-Einheiten. Das Signal wurde mittels eines Streulichtmessverfahrens bestimmt.

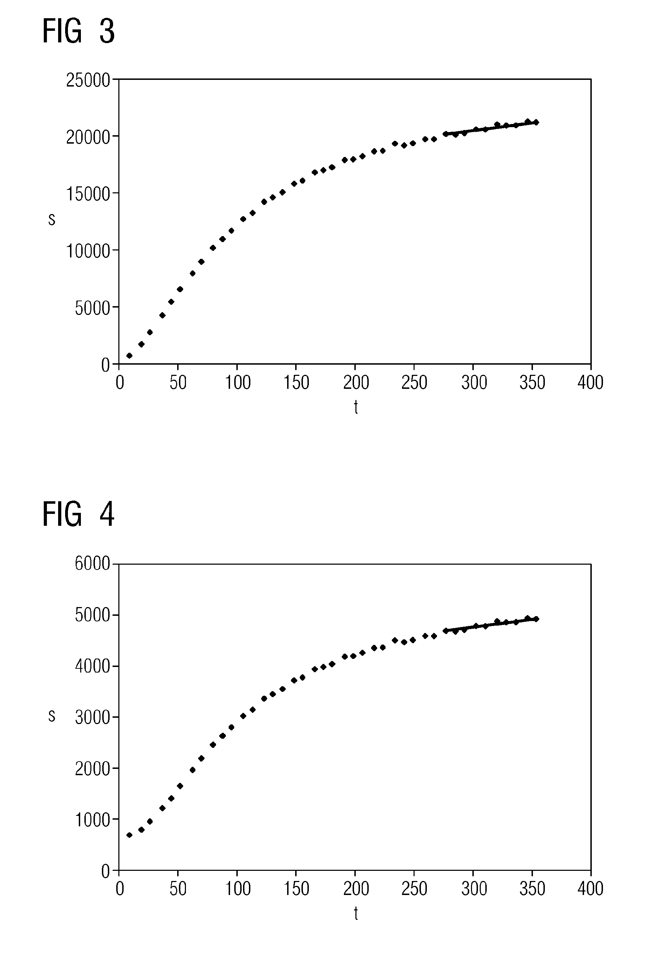

**Figur 3** zeigt den Verlauf einer mittels eines Streulichtmessverfahrens bestimmten Signal-Zeit-Kurve mit einzelnen Messpunkten einer Antigen-Antikörper-Reaktion eines Agglutinationsverfahrens zur Bestimmung der Immunglobulin A Konzentration in einer Probe mit einer Immunglobulin A Konzentration unterhalb der Konzentrationsschwelle ab der der High Dose Hook Effekt auftritt.
Illustriert ist wie die Steigung der Signal-Zeit-Kurve im Bereich von 277 bis 354 Sekunden mittels einer linearen Regressionsrechunung ermittelt wird.

**Figur 4** zeigt den Verlauf einer mittels eines Streulichtmessverfahrens bestimmten Signal-Zeit-Kurve mit einzelnen Messpunkten einer Reaktion zur Bestimmung der Immunglobulin-Leichtkette Typ Kappa Konzentration in einer Probe mit einer Immunglobulin-Leichtkette Typ Kappa Konzentration unterhalb der Konzentrationsschwelle ab der der High Dose Hook Effekt auftritt. Illustriert ist wie die Steigung der Signal-Zeit-Kurve im Bereich von 277 bis 354 Sekunden mittels einer linearen Regressionsrechunung ermittelt wird.

Gleiche Teile sind in allen Figuren mit denselben Bezugszeichen versehen.

### BEISPIELE

Die folgenden Ausführungsbeispiele dienen der Veranschaulichung der Erfindung und sind nicht als Einschränkung zu verstehen.

### BEISPIEL 1

In einer bevorzugten Ausführung kann das erfindungsgemäße Verfahren beispielsweise bei der quantitativen nephelometrischen Bestimmung von Immunglobulin A in Humanserum und Heparinplasma mittels Immunassay eingesetzt werden. Die Bestimmung von Immunglobulin A hilft bei der Diagnose eines gestörten Proteinstoffwechsels und einer verminderten Widerstandskraft gegen infektiöse Erreger.

Immunglobuline werden von Plasmazellen als humorale Immunantwort auf einen Kontakt des Immunsystems mit Antigenen gebildet. Bei Erstkontakt werden als Primärreaktion zunächst Antikörper der Klasse Immunglobulin M erzeugt, denen die Bildung von Immunglobulin G- und auch Immunglobulin A-Antikörpern folgt. Die quantitative Bestimmung der Immunglobuline kann wichtige Hinweise auf den humoralen Immunstatus liefern. Erniedrigte Immunglobulinkonzentrationen treten bei primären Immunmangelzuständen, z. B. selektivem Immunglobulin A-Mangel, der häufigsten primären Immundefizienzerkrankung, sowie bei sekundären Immuninsuffizienzen auf, z. B. bei fortgeschrittenen malignen Tumoren, lymphatischer Leukämie, multiplem Myelom und Morbus Waldenström. Erhöhte Immunglobulinkonzentrationen im Serum treten aufgrund polyklonaler oder oligoklonaler Immunglobulinvermehrung bei z. B. Lebererkrankungen (Hepatitis, Leberzirrhose), akuten und chronischen Infektionen, Autoimmunerkrankungen sowie bei Neugeborenen im Nabelschnurblut bei intrauterinen und perinatalen Infektionen auf. Monoklonale Immunglobulinvermehrungen im Serum sind z. B. bei Plasmozytomen, Morbus Waldenström und Schwerketten-Erkrankungen festzustellen.

Nach einer initialen Probenverdünnung im Verhältnis 1 zu 20 wird die Probe mit Reaktionspuffer beinhaltend eine Lösung von Polyethylenglycol und Natriumchlorid (11,6 g/L) in PhosphatPuffer (0,05 mol/L) mit Konservierungsmittel Natriumazid (< 1 g/L), und Antiserum gegen human Immunglobulin A vermischt und die Immunglobulin A abhängige Antigen-Antikörper-Reaktion in Gang gesetzt. Bevorzugt beträgt die Temperatur etwa 37 Grad Celsius und bevorzugt beträgt die Reaktionszeit etwa sechs Minuten, während der Streulichtintensität mittels einer nephelometrischen Methode mit Licht der Wellenlänge 840 nm gemessen wird. Bevorzugt wird die Streulichtintensität zu den Zeitpunkten 277, 285, 293, 303, 310, 321, 328, 336, 346, 354, jeweils in Sekunden nach in Gang Setzung der Immunglobulin A abhängigen Antigen-Antikörper-Reaktion ausgewertet und die korrespondierende Steigung der Signal-Zeit-Kurve mittels eines linearen Regressions-Algorithmus ermittelt und abgespeichert. Analog erfolgt die Ermittlung und Abspeicherung der Steigung der Referenzprobe mit einer Immunglobulin A Konzentrationen unterhalb der Schwelle zum Eintritt des High Dose Hook Effekts, bzw. die Ermittlung der Steigung der Referenzprobe mit einer Immunglobulin A Konzentrationen oberhalb der Schwelle zum Eintritt des High Dose Hook Effekts.

Zur Bestimmung der Konzentration von Immunglobulin A wird die Streulichtintensität zu den Zeitpunkten 9, 19, 26, 36, 44, 52, 62, 69, 80, 87, 95, 105, 112, 123, 130, jeweils in Sekunden nach in Gang Setzung der Immunglobulin A abhängigen Antigen-Antikörper-Reaktion ermittelt, und mittels eines linearen Regressions-Algorithmus der Wert der Streulichtintensität zum Zeitpunkt 8 Sekunden nach in Gang Setzung der Immunglobulin A abhängigen Antigen-Antikörper-Reaktion berechnet. Analog erfolgt die Ermittlung des Werts der Streulichtintensität zum Zeitpunkt 360 Sekunden nach in Gang Setzung der Immunglobulin A abhängigen Reaktion durch lineare Extrapolation der Werte der Streulichtintensität, die zu den Zeitpunkten 277, 285, 293, 303, 310, 321, 328, 336, 346, 354, jeweils in Sekunden nach in Gang Setzung der Immunglobulin A abhängigen Antigen-Antikörper-Reaktion ausgewertet wurden. Die Differenz der Werte der Streulichtintensität zum Zeitpunkt 8 Sekunden und zum Zeitpunkt 360 Sekunden wird ermittelt und als Maß für die Konzentration von Immunglobulin A in der Probe herangezogen.

Die ermittelte Steigung der Signal-Zeit-Kurve im Zeitraum 277 bis 354 Sekunden nach in Gang Setzung der Immunglobulin A abhängigen Antigen-Antikörper-Reaktion wird mit der Steigung der Referenzprobe mit einer definierten Analytkonzentration oder Analytaktivität unterhalb der Schwelle zum Eintritt des High Dose Hook Effekts verglichen. Das Vorliegen des High Dose Hook Effekts ist ausgeschlossen oder unwahrscheinlich, wenn die Steigung der Signal-Zeit-Kurve im Zeitraum 277 bis 354 Sekunden nach in Gang Setzung der Immunglobulin A abhängigen Antigen-Antikörper-Reaktion gleich oder kleiner ist als die Steigung der Referenzprobe mit einer definierten Immunglobulin A Konzentration unterhalb der Schwelle zum Eintritt des High Dose Hook Effekts.

Der überdurchschnittlich ansteigende Startbereich der Immunglobulin A abhängigen Antigen-Antikörper-Reaktion beginnt hier beispielsweise, wie in Figur 3 gezeigt, 30 Sekunden nach in Gang Setzung der Immunglobulin A abhängigen Antigen-Antikörper-Reaktion und endet 2 Minuten nach in Gang Setzung der Immunglobulin A abhängigen Antigen-Antikörper-Reaktion. Die Sättigung bzw. Nahezu-Sättigung der Immunglobulin A abhängigen Antigen-Antikörper-Reaktion wird hier beispielsweise nach 6 Minuten nach in Gang Setzung der Immunglobulin A abhängigen Antigen-Antikörper-Reaktion erreicht.

### BEISPIEL 2

In einer weiteren bevorzugten Ausführung kann das erfindungsgemäße Verfahren beispielsweise bei der quantitativen nephelometrischen Bestimmung von Immunglobulin-Leichtkette Typ Kappa in Humanserum, Humanplasma oder Urin eingesetzt werden. Bestimmungen von Immunglobulin-Leichtkette Typ Kappa unterstützt die Diagnose des Multiplen Myeloms (Plasmazelltumor), maligner Lymphome (Proliferation von Zellen des lypmphatischen Gewebes), der Waldenströms Makroglobulinämie (Proliferation von hochmolekularen Immunglobulinen [IgM]) und von Kollagenosen (systemischer Bindegewebsveränderungen) wie rheumatoider Arthrithis oder Systemischer Lupus erythematosus.

Die Konzentration von Immunglobulin-Leichtketten im Serum kann durch die Konzentration der kompletten Immunglobulin-Moleküle bestimmt werden, im Normalfall durch die Konzentration von Immunglobulin G, Immunglobulin A und Immunglobulin M. Abweichungen von diesem Zusammenhang treten auf, wenn freie Leichtketten oder freie Schwerketten im Serum vorliegen. Außer in diesen Fällen sind erhöhte oder erniedrigte Konzentrationen von Immunglobulin-Leichtketten durch Vermehrung (polyklonaler oder monoklonaler Natur) bzw. Verminderung der kompletten Immunglobuline bedingt. Während polyklonale Immunglobuline die beiden Leichtketten-Typen Kappa (K) und Lambda (L) im etwa konstanten Verhältnis von 2:1 aufweisen, besitzen monoklonale Immunglobuline nur einen Leichtketten-Typ (K oder L). Die vermehrte Produktion monoklonaler Immunglobuline oder monoklonaler freier Leichtketten kann daher zu einer Änderung des Leichtkettenquotienten K/L führen. Ein außerhalb des Referenzbereichs liegender K/L-Quotient ist somit ein Indiz für das Bestehen einer monoklonalen Gammopathie. Während komplette Immunglobulin-Moleküle die intakte glomeruläre Filtrationsbarriere nicht passieren können, werden freie Immunglobulin-Leichtketten glomerulär filtriert und tubulär reabsorbiert. Erhöhte Konzentrationen freier Immunglobulin-Leichtketten, wie sie z. B. von monoklonalen Plasmazellen freigesetzt werden, können die tubuläre Reabsorptionskapazität übersteigen und zur Ausscheidung
der freien Immunglobulin-Leichtketten im Urin führen. Der Nachweis freier Immunglobulin-Leichtketten im Urin (= Bence-Jones-Proteine) ist somit ein wichtiger Hinweis für das Vorliegen monoklonaler Gammopathien, was sowohl für die Diagnostik als auch für die Verlaufskontrolle genutzt werden kann.

Nach einer initialen Probenverdünnung im Verhältnis 1 zu 20 wird die Probe mit Reaktionspuffer beinhaltend eine Lösung von Polyethylenglycol und Natriumchlorid (11,6 g/L) in PhosphatPuffer (0,05 mol/L) mit Konservierungsmittel Natriumazid (< 1 g/L), und Antiserum gegen human Immunglobulin-Leichtkette Typ Kappa vermischt und die Immunglobulin-Leichtkette Typ Kappa abhängige Antigen-Antikörper-Reaktion in Gang gesetzt. Bevorzugt beträgt die Temperatur etwa 37 Grad Celsius und bevorzugt beträgt die Reaktionszeit etwa sechs Minuten während der Streulichtintensität mittels einer nephelometrischen Methode mit Licht der Wellenlänge 840 nm gemessen wird. Bevorzugt wird die Streulichtintensität zu den Zeitpunkten 277, 285, 293, 303, 310, 321, 328, 336, 346, 354, jeweils in Sekunden nach in Gang Setzung der Immunglobulin-Leichtkette Typ Kappa abhängigen Antigen-Antikörper-Reaktion ausgewertet, und die korrespondierende Steigung der Signal-Zeit-Kurve mittels eines linearen Regressions-Algorithmus ermittelt und abgespeichert. Analog erfolgt die Ermittlung und Abspeicherung der Steigung der Referenzprobe mit einer Immunglobulin-Leichtkette Typ Kappa Konzentrationen unterhalb der Schwelle zum Eintritt des High Dose Hook Effekts bzw. die Ermittlung der Steigung der Referenzprobe mit einer Immunglobulin-Leichtkette Typ Kappa Konzentrationen oberhalb der Schwelle zum Eintritt des High Dose Hook Effekts.

Zur Bestimmung der Konzentration von Immunglobulin-Leichtkette Typ Kappa wird die Streulichtintensität zu den Zeitpunkten 9, 19, 26, 36, 44, 52, 62, 69, 80, 87, 95, 105, 112, 123, 130, jeweils in Sekunden nach in Gang Setzung der Immunglobulin-Leichtkette Typ Kappa abhängigen Antigen-Antikörper-Reaktion ermittelt, und mittels eines linearen Regressions-Algorithmus der Wert der Streulichtintensität zum Zeitpunkt 8 Sekunden nach in Gang Setzung der Immunglobulin-Leichtkette Typ Kappa abhängigen Antigen-Antikörper-Reaktion berechnet. Analog erfolgt die Ermittlung des Werts der Streulichtintensität zum Zeitpunkt 360 Sekunden nach in Gang Setzung der Immunglobulin-Leichtkette Typ Kappa abhängigen Antigen-Antikörper-Reaktion durch lineare Extrapolation der Werte der Streulichtintensität, die zu den Zeitpunkten 277, 285, 293, 303, 310, 321, 328, 336, 346, 354, jeweils in Sekunden nach in Gang Setzung der Immunglobulin-Leichtkette Typ Kappa abhängigen Antigen-Antikörper-Reaktion ausgewertet wurden. Die Differenz der Werte der Streulichtintensität zum Zeitpunkt 8 Sekunden und zum Zeitpunkt 360 Sekunden wird ermittelt und als Maß für die Konzentration von Immunglobulin-Leichtkette Typ Kappa in der Probe herangezogen.

Die ermittelte Steigung der Signal-Zeit-Kurve im Zeitraum 277 bis 354 Sekunden nach in Gang Setzung der Immunglobulin-Leichtkette Typ Kappa abhängigen Antigen-Antikörper-Reaktion wird mit der Steigung der Referenzprobe mit einer definierten Analytkonzentration oder Analytaktivität unterhalb der Schwelle zum Eintritt des High Dose Hook Effekts verglichen. Das Vorliegen des High Dose Hook Effekts ist ausgeschlossen oder unwahrscheinlich, wenn die Steigung der Signal-Zeit-Kurve im Zeitraum 277 bis 354 Sekunden nach in Gang Setzung der Immunglobulin-Leichtkette Typ Kappa abhängigen Antigen-Antikörper-Reaktion gleich oder kleiner ist als die Steigung der Referenzprobe mit einer definierten Immunglobulin-Leichtkette Typ Kappa Konzentration unterhalb der Schwelle zum Eintritt des High Dose Hook Effekts.

Der überdurchschnittlich ansteigende Startbereich der Immunglobulin-Leichtkette Typ Kappa abhängigen Antigen-Antikörper-Reaktion beginnt hier beispielsweise, wie in Figur 4 gezeigt, 30 Sekunden nach in Gang Setzung der Immunglobulin-Leichtkette Typ Kappa abhängigen Antigen-Antikörper-Reaktion und endet 2 Minuten nach in Gang Setzung der Immunglobulin-Leichtkette Typ Kappa abhängigen Antigen-Antikörper-Reaktion. Die Sättigung bzw. Nahezu-Sättigung der Immunglobulin-Leichtkette Typ Kappa abhängigen Antigen-Antikörper-Reaktion wird hier beispielsweise nach 6 Minuten nach in Gang Setzung der Immunglobulin-Leichtkette Typ Kappa abhängigen Antigen-Antikörper-Reaktion erreicht.

Die erfindungsgemäßen Verfahren und Gegenstände der vorliegenden Patentanmeldung werden außerdem in den Patentansprüchen beschrieben.

**Bezugszeichenliste**

| | |
|---|---|
| S | Signal |
| t | Zeit in Sekunden |

## Patentansprüche

1. Verfahren zur Bestimmung der Konzentration oder Aktivität eines Analyten in einer Probe, das folgende Schritte umfasst:
a. Vermischen der Probe mit mindestens einem Reagenz, wodurch eine analytabhängige Reaktion in Gang gesetzt wird;
b. Messung eines sich infolge der analytabhängigen Reaktion ändernden Signals (x) über die Zeit (t);
c. Bestimmung der Steigung A der Signal-Zeit-Kurve in mindestens einem Zeitintervall;
d. Vergleich der Steigung A mit der ermittelten Steigung der Signal-Zeit-Kurve einer Referenzprobe mit einer definierten Analytkonzentration oder Analytaktivität oberhalb der Schwelle zum Eintritt des High Dose Hook Effekts, und /oder mit der ermittelten Steigung der Signal-Zeit-Kurve einer Referenzprobe mit einer definierten Analytkonzentration oder Analytaktivität unterhalb der Schwelle zum Eintritt des High Dose Hook Effekts;
**dadurch gekennzeichnet, dass**
die Bestimmung der Steigung A der Signal-Zeit-Kurve innerhalb eines Zeitraums beginnend 2 Minuten und endend 30 Minuten nach in Gangsetzung der analytabhängigen Reaktion durch Vermischen der Probe mit mindestens einem Reagenz erfolgt, und wobei
i) das Vorliegen des High Dose Hook Effekts verifiziert oder wahrscheinlich ist, wenn die Steigung A der Signal-Zeit-Kurve gleich oder größer ist als die Steigung der Referenzprobe mit einer definierten Analytkonzentration oder Analytaktivität oberhalb der Schwelle zum Eintritt des High Dose Hook Effekts, und / oder
ii) das Vorliegen des High Dose Hook Effekts ausgeschlossen oder unwahrscheinlich ist, wenn die Steigung A der Signal-Zeit-Kurve gleich oder kleiner ist als die Steigung der Referenzprobe mit einer definierten Analytkonzentration oder Analytaktivität unterhalb der Schwelle zum Eintritt des High Dose Hook Effekts.

2. Verfahren nach Anspruch 1 **dadurch gekennzeichnet, dass** die Bestimmung der Steigung A der Signal-Zeit-Kurve innerhalb eines Zeitraums von 3 Minuten bis 20 Minuten, bevorzugt von 4 bis 10 Minuten, besonders bevorzugt von 4 bis 7 Minuten, ganz besonders bevorzugt von 5 bis 6 Minuten nach in Gangsetzung der analytabhängigen Reaktion durch Vermischen der Probe mit mindestens einem Reagenz erfolgt.

3. Verfahren gemäß einem der vorhergehenden Ansprüche, **dadurch gekennzeichnet, dass** die Steigung A der Signal-Zeit-Kurve mit Hilfe einer linearen Regressionsrechnung bestimmt wird.

4. Verfahren gemäß einem der vorhergehenden Ansprüche, wobei die Konzentration oder Aktivität eines Analyten in einer biologischen Probe aus der Gruppe Vollblut, Plasma, Serum, Urin, Faeces, Speichel oder Liquor bestimmt wird.

5. Verfahren gemäß einem der vorhergehenden Ansprüche, wobei die analytabhängige Reaktion eine Antigen-Antikörper-Reaktion ist.

6. Verfahren gemäß einem der vorhergehenden Ansprüche, wobei ein Signal aus der Gruppe Streulicht, Trübung, Fluoreszenz und Chemilumineszenz gemessen wird.

7. Verfahren gemäß einem der vorhergehenden Ansprüche zur Bestimmung der Konzentration von Immunglobulin A oder Immunglobulin-Leichtkette Typ Kappa.

8. **Gerät** zur Durchführung eines Verfahrens gemäß einem der Ansprüche 1 - 7 zur Bestimmung der Konzentration oder Aktivität eines Analyten in einer Probe.

9. **Gerät** gemäß Anspruch 8 **dadurch gekennzeichnet, dass** es Mittel zur Steuerung der Durchführung der Verfahrensschritte des Verfahrens gemäß einem der Ansprüche 1-7 zur Bestimmung der Konzentration oder Aktivität eines Analyten in einer Probe umfassst.

10. **Speichermedium dadurch gekennzeichnet, dass** ein Programm zur Steuerung der Durchführung eines Verfahrens zur Bestimmung der Konzentration oder Aktivität eines Analyten in einer Probe gemäß einem der Ansprüche 1 - 7 darauf abgespeichert ist.
